# EUROPEAN PATENT SPECIFICATION

(11) **EP 2 739 256 B1**
(45) Date of publication and mention of the grant of the patent: **04.05.2016**
(21) Application number: 12737285.2
(22) Date of filing: 19.07.2012
(51) Int. Cl.: A61J 1/06, A61M 5/24, A61M 5/31, A61M 5/315

(54) **DRUG DELIVERY DEVICE AND CARTRIDGE TO BE INTERCONNECTED THEREWITH**
WIRKSTOFFFREISETZUNGSVORRICHTUNG UND DARAN ANZUSCHLIESSENDE KARTUSCHE
DISPOSITIF D'ADMINISTRATION DE MÉDICAMENT ET CARTOUCHE À RELIER À CELUI-CI

(30) Priority: 02.08.2011 EP 11176238
(43) Date of publication of application: 11.06.2014
(73) Proprietor: Sanofi-Aventis Deutschland GmbH, 65929 Frankfurt am Main (DE)
(72) Inventor: HOPPE, Hendrik, 65926 Frankfurt (DE); JOSEF, Clemens, 65926 Frankfurt am Main (DE)
(74) Representative: Weilnau, Carsten
(86) International application number: PCT/EP2012/064154
(87) International publication number: WO 2013/017415

(56) References cited:
- EP-A2- 0 937 473
- WO-A1-00/02605
- WO-A1-03/047665
- WO-A1-2011/068544

## Description

### Field of the Invention

The present invention relates to the field of drug delivery devices and in particular to injection devices such like pen-type injectors for administering a predefined dose of a liquid medicament.

### Background and Prior Art

Drug delivery devices allowing for multiple dosing of a required dosage of a liquid medicament, such as liquid drugs, and further providing administration of the medicament to a patient, are as such well-known in the art. Generally, such devices have substantially the same purpose as that of an ordinary syringe.

Drug delivery devices of this kind have to meet a number of user specific requirements. For instance in case of those with diabetes, many users will be physically infirm and may also have impaired vision. Therefore, these devices need to be robust in construction, yet easy to use, both in terms of the manipulation of the parts and understanding by a user of its operation. Further, the dose setting must be easy and unambiguous and where the device is to be disposable rather than reusable, the device should be inexpensive to manufacture and easy to dispose. In order to meet these requirements, the number of parts and steps required to assemble the device and an overall number of material types the device is made from have to be kept to a minimum.

Typically, the medicament to be administered is provided in a cartridge having a moveable piston or bung mechanically interacting with a piston rod of a drive mechanism of the drug delivery device. By applying thrust to the piston in a distal direction, a predefined amount of the medicament fluid is expelled from the cartridge.

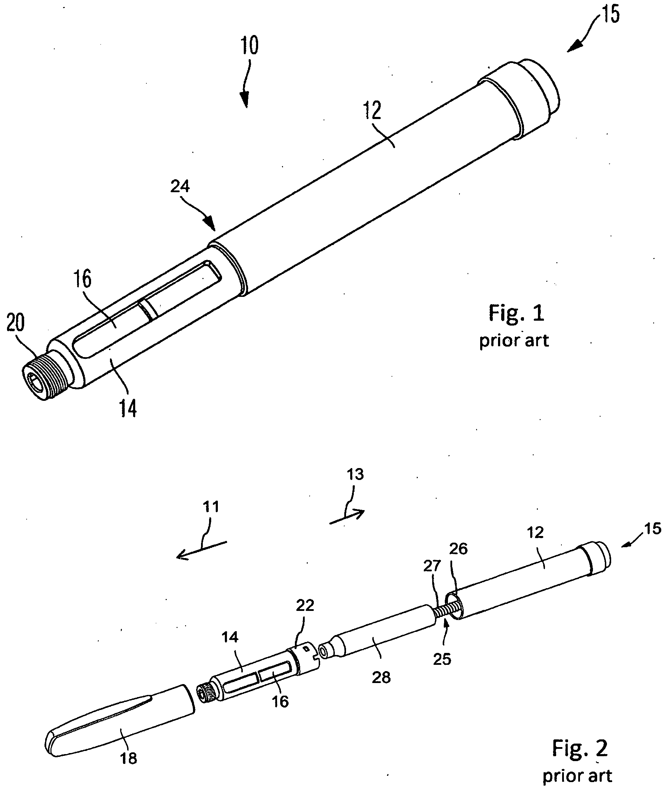

In particular for elderly or physically infirm users, the overall handling of the device in a home medication environment should be simple and highly reliable. As for instance illustrated in Figures 1 and 2, drug delivery devices and in particular pen-type injectors typically comprise a multi-component housing 10. Here, a distal end section typically serving as a cartridge holder 14 is interconnected with a proximal housing component, denoted as body 12. The cartridge holder 14 comprises a threaded socket 20 at its distal end to receive a needle assembly being not explicitly illustrated here, that has a correspondingly threaded needle hub and a double tipped injection needle.

The cartridge holder 14 further comprises an insert portion 22 at its proximal end, by way of which the cartridge holder 14 can be at least partially inserted into a correspondingly shaped distal receptacle 26 of the proximal housing component 12 of the drug delivery device. This way an interface 24 between cartridge holder 14 and body 12 can be provided. The body 12 serves to accommodate a drive mechanism 25 having a piston rod 27 to become operably engaged with a piston of a cartridge 28 which is to be disposed and fixed in the cartridge holder 14. By way of an inspection window 16, the fluid or filling level of the cartridge 28 can be visually inspected.

No matter on whether the illustrated device 10 is of reusable or disposable type, such pen-type injectors 10 are generally manufactured and assembled in a mass production line. There, the supply of the individual components, the device is assembled of is a key factor for the efficiency of the manufacturing and assembly process as well as for the quality and reliability of the drug delivery devices itself. In particular, since the cartridges filled with the medicament are typically made of glass, there exists a certain danger of fracture or breakage, especially, when a large quantity of filled cartridges is transported or otherwise handled in the production or assembly process.

With reusable devices, wherein empty cartridges are replaced by filled ones the drug delivery device can be assembled without a cartridge initially arranged therein. However, with disposable devices, intended to be entirely discarded upon consumption of the medicament, the cartridge itself has to be arranged and properly positioned in the device during the device assembly process.

Especially with such disposable devices, the cartridge 28 is arranged in the cartridge holder 14, which thereafter is to be non-releasably and permanently connected with the body 12 of the drug delivery device 10. During or prior to this final assembly step, wherein cartridge holder 14 and body 12 are interconnected, the cartridge 28 can become subject to inadmissible point loads, that may damage the cartridge 28.

Moreover, in such mass production processes, even the pre-assembly of e.g. arranging the cartridge 28 inside the cartridge holder 14 and/or other steps of e.g. positioning and orienting of a piston inside a cartridge 28 have to be separately controlled at the end of respective pre-assembly steps.

Document WO 2011/068544 A1 discloses a cartridge having a connection thread for connecting a pen needle assembly directly to the cartridge body, which body may be made of a transparent plastic material.

Document EP 0 937 473 A2 discloses a cartridge body including a raised triangular ring on an outer surface at a proximal end of the cartridge body, which is received by a triangular recess on an inner wall within a pen body housing.

Document WO 00/02605 A1 discloses a cartridge assembly having coupling means for engaging a needle assembly and/or for engaging a dosing assembly. At least one of said coupling means is unitarily molded with the cartridge. The coupling means may further be selected from threaded locks, snap locks, hinged locks or bayonet locks.

The multiplicity of functional components, such drug delivery devices are made of therefore requires respective control steps and mechanisms in an industrial mass production process.

### Objects of the Invention

It is therefore an object of the present invention to provide a drug delivery device allowing for and supporting a simplified assembly particularly suitable for mass production processes. By way of such a modified and assembly-optimized drug delivery device, the process reliability of a mass production process should be enhanced. It is a further aim to simplify the structure of drug delivery devices and to reduce production costs and respective expenditures, particularly in terms of component supply and logistics.

### Summary of the Invention

In one aspect, the invention relates to a cartridge for a drug delivery device and in particular to a cartridge intended to cooperate with a drive mechanism of a drug delivery device, such as a pen-type injector. The cartridge comprises a barrel of substantially cylindrical or tubular shape which is sealed by a piston slidably displaced in the barrel along an axial direction. Typically, the piston is initially located near a proximal end of the barrel and is intended to be stepwise displaced in distal direction for expelling respective pre-defined doses of the liquid medicament contained and stored in the cartridge.

The cartridge at its distal end section typically has an outlet and is sealed by a pierceable seal, like a septum, which is typically arranged and fixed at the outlet section of the cartridge by way of the beaded cap. The distal seal or septum is intended and designed to be pierced and penetrated by a piercing assembly like a double-tipped injection needle.

The cartridge and/or its barrel further comprises an interface portion near a proximal end to engage with a body of the drug delivery device. By way of the interface portion typically provided in or on the barrel of the cartridge, a direct mutual interconnection between body and cartridge can be established.

Moreover, the barrel comprises at least one radially extending portion at its outer periphery and in a midsection of the barrel to cooperate with at least one further functional component of the device. The functional component of the device may be designed as a removable cap in order to cover and/or to protect the cartridge. Moreover, the functional component may also be represented by additional cartridges, especially when multiple cartridges are stored or stacked in a densely packed configuration in a mass transport container.

The radially extending portion comprises an annular protrusion which is flattened and comprises an outer surface extending parallel to the longitudinal axis of the cartridge.

Such protruding portions may facilitate general handling and gripping of the cartridge, e.g. in a mass production environment. In this context it is further conceivable, that the barrel comprises several annular recesses or annular protruding portions or rims being for instance regularly arranged along the barrel's long axis. When the barrel comprises a multiplicity of recesses and/or protruding portions, radial extension of recesses or protruding portions may be always identical or may feature a radially staggered structure in order to match with the correspondingly shaped functional components.

In a further aspect, the annular protruding portion serves as a peripheral support face to enable mutual peripheral abutment between a plurality of cartridges, e.g. densely arranged in or on a transport container. By way of mutually corresponding peripheral support faces, the cartridges become less susceptible to fracture and damage when densely packed in or on a transport container. Moreover, when several cartridges filled with the medicament are delivered to end consumers, packaging density in a blister packaging or in boxes, wherein adjacently arranged cartridges may get in direct contact with each other, may be increased and a package containing numerous cartridges may become more robust and less sensitive to mechanical load, shock or other types of impact.

According to another aspect, the interface of cartridge and/or barrel comprises a bayonet catch mechanism and/or a latching assembly, by way of which a releasable and/or non-releasable interconnection of cartridge and body of the drug delivery device can be easily and reliably established. Depending on whether the drug delivery device is designed as a reusable or disposable device, the bayonet catch mechanism and/or latching assembly is either of releasable or non-releasable type. In case a non-releasable type interface is required for a disposable drug delivery device, the interface is preferably provided with self-destructing means, that serve to at least partially destroy the interface portion of the body in order to inhibit unintentional replacement of empty cartridges or comparable misuse of the device.

According to a further embodiment, the barrel comprises at least one annular recess and/or at least one annular protruding portion at its outer periphery. This way, the radially extending portion is of annular structure. With such a circular symmetric protruding portion or recess, the cartridge can always be handled and gripped or positioned in a transport container irrespective of its orientation with respect to its longitudinal symmetry axis. The recessed or protruding portion of the barrel may comprise one or several circumferential rims.

In a further preferred embodiment, the cartridge comprises a threaded portion near its distal end to engage with a needle assembly. Preferably, the cartridge has a stepped down neck portion or socket covered by a beaded cap in order to arrange and to fix a pierceable sealing member at a distal outlet of the barrel. The threaded portion is preferably provided at a distal end of a substantially cylindrically shaped portion of the barrel. Instead of a threaded portion, e.g. designed as an outer thread, it is also conceivable to provide other types of fixing or mounting means at the distal end of the barrel, which may provide a snap-fit or latching connection.

In a further aspect, the piston slidably arranged in the barrel is of symmetric shape in axial direction. Hence, a proximal and a distal end face as well as proximally and distally located sealing ribs of the piston are substantially identically shaped. This way, the piston can be arranged inside the barrel in different arbitrary configurations. As a consequence, during assembly or filling of the cartridge, the orientation of the piston displaced therein does no longer have to be controlled.

In still another aspect, the cartridge comprises a barrel which is entirely made of a plastic material. For instance, the plastic material may comprise a polymeric material or polycarbonate. The plastic material is substantially inert to the medicament provided in the cartridge. Moreover, the inside facing walls of the barrel may be provided with an inert coating.

Additionally it is to be noted, that the radially extending structure or recess at the outer periphery of the barrel may specify a standardized geometric pattern allowing to mechanically encode and to identify cartridges of different types that are to be used with appropriately designed and different drug delivery devices. Moreover, the modified periphery of the barrel and/or its geometric design may also provide and specify a standardized cartridge design being applicable to a large variety of different drug delivery devices or comparable dispensing arrangements.

The present invention also relates to a drug delivery device for setting and dispensing a dose of a liquid medicament. The device, typically designed as a pen-type injector comprises a body to accommodate a drive mechanism having at least a piston rod, which is typically to be driven in distal direction in order for exerting respective distally directed thrust to a piston of a cartridge. The device further comprises a cartridge having a substantially cylindrical barrel which is typically sealed by a piston in a proximal direction. The piston is slidably displaced in the barrel along an axial direction. By exerting distally directed pressure to the piston, e.g. by way of the driven piston rod, a respective fluid pressure inside the cartridge may built-up, thereby expelling a pre-defined dose of the liquid medicament from the cartridge.

Moreover, the cartridge itself is directly connectable with the body by way of an interface and the barrel further comprises at least one radially extending portion at its outer periphery or circumference to cooperate with at least one further functional component of the device. By providing an interface, the cartridge can be directly connected with the proximally located body. An additional cartridge holder therefore becomes rather superfluous. Hence, by way of the interface between cartridge and body, the functionality of a cartridge holder can be entirely incorporated or embedded in the cartridge itself.

As a consequence, the number of parts, the drug delivery device is assembled of can be advantageously reduced. Additionally, the pre-assembling procedure in a mass production line can be simplified. Hence, a relative position of cartridge and cartridge holder does no longer have to be controlled in the course of device assembly.

The barrel is of substantially cylindrical shape with its cylinder long axis extending in axial direction. The outer periphery, hence the mantel- or lateral surface of the barrel further comprises at least one radially extending portion which is intended to mechanically interact with additional functional components of the device. Such functional components may be for instance a removable cap or a protective sheath being intended to protect the cartridge, hence the distal portion of the drug delivery device, especially when not in use.

However, also the cartridge itself may serve as a functional component in the present context. The radially extending portion then serves as an abutment or support structure, by way of which a rather large number of individual cartridges can be densely packed, e.g. in a mass tray arrangement prior to or during the manufacturing or assembly process. By way of the at least one radially extending portion provided at the outer periphery of the barrel, the various cartridges becomes less susceptible to fracture or damage when subject to external load or mechanical impact.

Additionally, by way of the at least one radially extending portion provided in a midsection of the barrel, a fastening, handling and/or gripping structure can be provided, which allows for a secure and reliable handling of the cartridges in a mass production process. Hence, the radially extending portion provided at the outer periphery of the barrel may provide a pre-defined gripping means, e.g. allowing a rather stress free, e.g. positive engaging gripping and overall handling of the cartridges, especially for fully- or semi-automated assembly processes.

According to a preferred embodiment, the barrel is adapted to engage with a removable cap which is designed to cover and/or to protect the cartridge. Here, the outer periphery of the barrel and a corresponding inner side wall structure of the cap comprise mutually corresponding or mutually cooperating fastening means, e.g. in form of snap- or clip features, that provide releasable and positive engagement of barrel and cap.

According to a further embodiment, the interface of cartridge and body is adapted to provide a releasable or non-releasable interconnection of cartridge and body. Depending on whether the drug delivery device is designed as a reusable or disposable device, the interface between cartridge and body is either of releasable or non-releasable type, respectively. Especially with a non-releasable interconnection to be provided with disposable devices, it is intended, that a brute-force disassembly of cartridge and body leads to a severe damaging at least of the body component, in order to disable and to prohibit unintentional replacement of an empty cartridge. It is also conceivable, that the mutual interconnection of cartridge and body is established and/or supported by way of adhesives or by means of a welding process.

According to a further aspect, the barrel and/or the entire cartridge is or are designed as an integral part or component of the housing of the device. Hence, the barrel of the cartridge therefore effectively replaces the cartridge holder component of the device housing.

In still another aspect, the drive mechanism accommodated and arranged in the proximally located body of the housing is adapted to set and/or to dispense a pre-defined dose of the medicament. Preferably, the drive mechanism comprises a dose dial as well as a dose button, by way of which the size of the dose can be individually manipulated by the end user. The dose button and/or dose dial may be further used to initiate or to conduct a manually operated or even a semi- or fully-automated dose dispensing action. The body of the drug delivery device typically comprises a tubular shaped housing component generally resembling the shape and geometry of the tubular shaped cartridge.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-pal*mitoyl-LysB28ProB29 human insulin; B30-N-myristoyi-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [lsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or
des Pro36 [Asp28] Exendin-4(1-39),
des Pro36 [lsoAsp28] Exendin-4(1-39),
des Pro36 [Met(O)14, Asp28] Exendin-4(1-39),
des Pro36 [Met(O)14, lsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(02)25, Asp28] Exendin-4(1-39),
des Pro36 [Met(O)14 Trp(02)25, lsoAsp28] Exendin-4(1-39),
   wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheet create a "sandwich" shape, held together by interactions between conserved cysteines and other charged amino acids.

There are five types of mammalian lg heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and E have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single lg domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the lg prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

It will be further apparent to those skilled in the pertinent art that various modifications and variations can be made to the present invention without departing from the spirit and scope of the invention. Further, it is to be noted, that any reference signs used in the appended claims are not to be construed as limiting the scope of the present invention.

### Brief Description of the Drawings

In the following preferred embodiments of the invention will be described by making reference to the drawings in which:
- Figure 1: shows a conventional drug delivery device according to the prior art in a perspective illustration,
- Figure 2: is illustrative of the drug delivery device according to Figure 1 in an exploded view,
- Figure 3: shows a cross section through a cartridge according to the present invention,
- Figure 4: is illustrative of a side view of the cartridge according to Figure 3, and
- Figure 5: separately illustrates a symmetric shaped piston of the cartridge according to Figure 3.

### Detailed Description

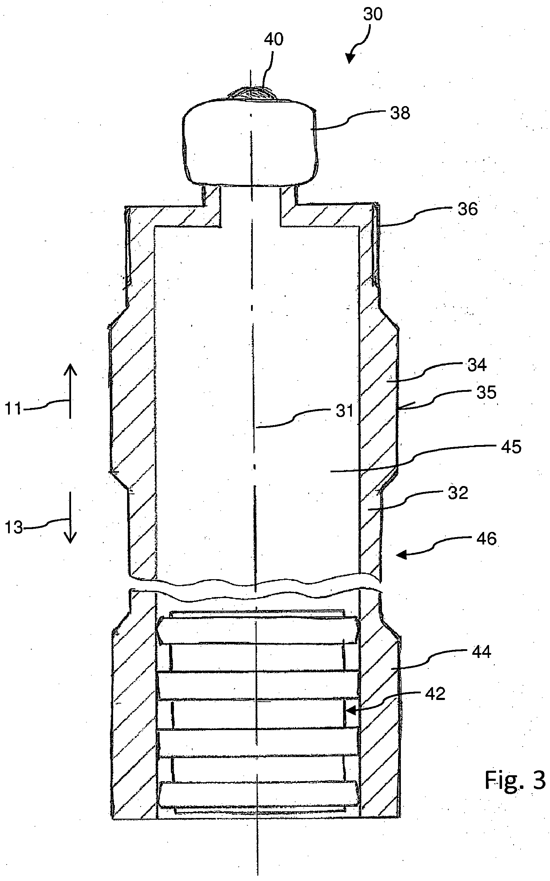

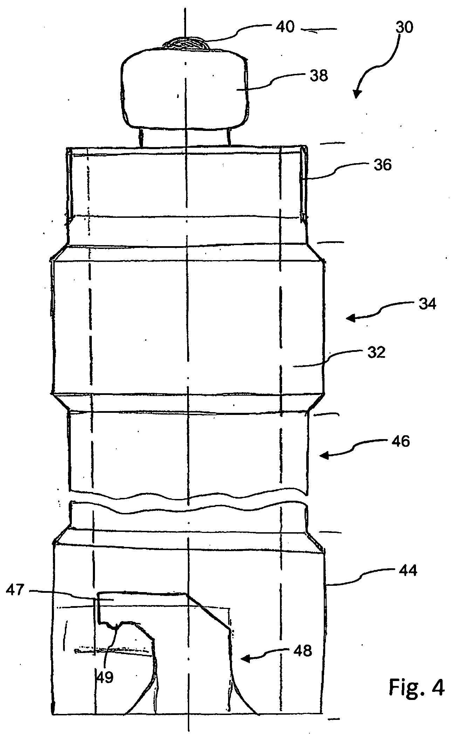

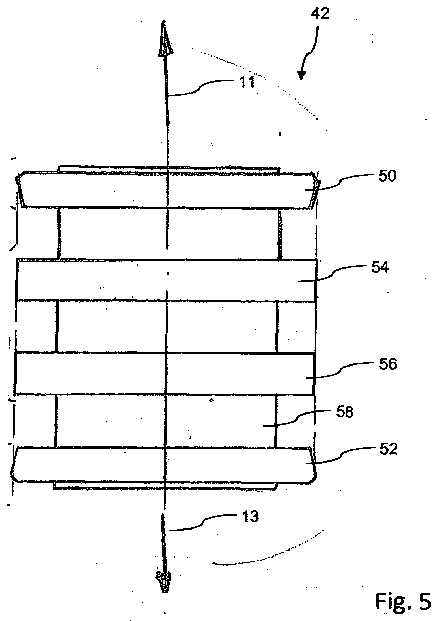

The cartridge 30 as illustrated in Figures 3 and 4 comprises a substantially tubular shaped barrel 32 being sealed in proximal direction 13 by way of a piston 42, which is separately illustrated in Figure 5. The barrel 32 of the cartridge 30 comprises a beaded cap 38 at a distal end portion by way of which a pierceable septum 40 can be kept in position thereon. A distal end of the rather cylindrically or tubular shaped barrel 32 is further provided with an outer thread 36 in order to threadedly receive a needle assembly or a needle hub being not further illustrated here.

The needle hub to be screwed onto the outer thread 36 of the barrel 32 is further equipped with a double tipped needle adapted to penetrate the distally located seal or septum 40 of the cartridge 30. With a needle assembly mounted on the cartridge 30, application of distally directed pressure to the piston 42 correspondingly establishes a fluid pressure inside the inner volume 45 of the cartridge 30, by way of which a pre-defined amount of the medicament contained in the barrel 32 can be dispensed via the needle assembly being in fluid connection therewith.

Along its outer periphery, the barrel 32 comprises at least one radially extending portion 34 designed as an annular protrusion and/or as an annular recess 46. The annular protruding portion 34 is flattened and comprises an outer surface 35 extending substantially parallel to the longitudinal axis or symmetry axis 31 of the cartridge 30. Since the radially extending portion 34 comprises a rather large peripheral contact surface 35, a multiplicity of identically shaped cartridges 30 can be arranged in a densely packed configuration, e.g. in a transport container with a reduced or negligible risk of fracture in case the barrel 32 comprises a vitreous body, e.g. made of glass.

Moreover, the at least one radially extending side wall portion 34 and/or the adjacently located recess 46 provide a well-defined handling structure for gripping and positioning the cartridge 30 in a mass production process. Radially inwardly directed gripping forces, typically exerted by automated assembly devices can be remarkably reduced due to the geometric structure 34, 46 of the outer periphery of the barrel 32. Additionally, the cartridge 30 can be mechanically encoded by the outer structure of the barrel 32. Also, the illustrated radial structures 34, 36 of the barrel 32 may define a standardized pattern, to be used with a large variety of different drug delivery devices.

Moreover, since the barrel 32 is directly provided with a distally located outer thread 36, the cartridge 30 also serves as an integral, part of the housing of the drug delivery device. As a consequence, a conventional cartridge holder 14 as illustrated in Figures 1 and 2 becomes superfluous and is no longer required. In effect, the cartridge 30 as illustrated in Figures 3 and 4 comprises an interface 48 near its proximal end as illustrated in the lower section of Figure 4. By way of the interface 48, which may comprise a bayonet catch mechanism and/or a latching or even screwing assembly, a direct interconnection of cartridge 30 and proximal housing component 12 of a drug delivery device can be attained.

This way, also mechanical and geometric as well as assembly tolerances can be reduced and effects of elastic deformation of device components can be minimized. Moreover, mechanical load across the device propagating in axial direction 11, 13 can be transferred to the piston 42 in a more direct and unadulterated way.

The interface 48 as depicted in Figure 4 comprises a bayonet-like catch mechanism. It comprises a bended receptacle 47 or slit adapted to receive a correspondingly shaped prong or protrusion provided at a respective interface section of the body of the housing. Moreover, near a dead end of the receptacle 47 there is provided a fixing groove 49 adapted to mate with a correspondingly shaped protrusion. This way, once assembled, mutually corresponding interface sections of cartridge 30 and body 12 will be hindered to release automatically.

The sketch of Figure 5 is further illustrative of a piston 42 adapted to initially seal the proximal portion 44 of the cartridge 30. The seal or piston 42 as shown in Figure 5 is symmetrically designed in distal direction 11 and in proximal direction 13. This way, the piston 42 may either be inserted into the barrel 32 as illustrated in Figure 5 or in an upside-down configuration. In distal and proximal direction 11, 13, the piston 42 comprises laterally or radially outwardly extending sealing ribs 50, 52 to but against an inside facing tubular shaped side wall of the barrel 32.

Between these axially outwardly arranged sealing lips or ribs 50, 52 there are provided two additional support ribs 54, 56 that serve as axial and radial guiding elements. By way of the two central ribs 54, 56, a tilting or skewing motion of the piston 42 inside the barrel 32 can be effectively prevented. The various radially protruding annular ribs 50, 52, 54, 56 are interconnected by a solid base portion 58 featuring a substantially cylindrical shape. The piston 42 is typically made of a natural or synthetic rubber material.

### Reference Numbers

- 10: drug delivery device
- 11: distal direction
- 12: body
- 13: proximal direction
- 14: cartridge holder
- 16: inspection window
- 18: protective cap
- 20: socket
- 22: mount
- 24: interface
- 25: drive mechanism
- 26: mount
- 27: piston rod
- 28: cartridge
- 30: cartridge
- 31: longitudinal axis
- 32: barrel
- 34: radially extending portion
- 35: surface portion
- 36: outer thread
- 38: bedded cap
- 40: septum
- 42: piston
- 44: proximal barrel section
- 45: inner volume
- 46: annular recess
- 47: receptacle
- 48: interface
- 49: fixing groove
- 50: sealing lip
- 52: sealing lip
- 54: support rib
- 56: support rib
- 58: base portion

## Claims

1. A cartridge for a drug delivery device for setting and dispensing a dose of a medicament and comprising a body (12) to accommodate a drive mechanism (25) having a piston rod (27), the cartridge comprising:
- a barrel (32) sealed by a piston (42) slidably displaced therein along an axial direction (11, 13),
- an interface portion (48) near a proximal end to engage with a body (12) of the drug delivery device (10), and
- wherein the barrel (32) comprises at least one radially extending portion (34) at its outer periphery to cooperate with at least one further functional component (30) of the device, and
- the radially extending portion (34) comprising an annular protrusion which is flattened and comprises an outer surface (35) extending parallel to the longitudinal axis (31) of the cartridge, **characterized in that** the radially extending portion is provided in a midsection of the barrel.

2. The cartridge according to claim 1, wherein the interface comprises a bayonet catch mechanism and/or a latching assembly.

3. The cartridge according to claim 1 or 2, wherein the barrel (32) comprises at least one annular recess (46) and/or at least one annular protruding portion (34) at its outer circumference.

4. The cartridge according to claim 3, wherein the annular protruding portion comprises a peripheral support face to enable mutual peripheral abutment between a plurality of cartridges densely arranged in or on a transport container.

5. The cartridge according to any one of the preceding claims 2 to 4, wherein the piston (42) is of symmetric shape in axial direction (11, 13).

6. The cartridge according to any one of the preceding claims 2 to 5, wherein the barrel (32) is made of a plastic material.

7. The cartridge according to any one of the preceding claims 2 to 6, wherein the interface (48) is adapted to provide a non-destructive and/or destructive interconnection between cartridge (30) and body (12).

8. A drug delivery device for setting and dispensing a dose of a medicament, comprising:
- a body (12) to accommodate a drive mechanism (25) having a piston rod (27), and
- a cartridge (30) according to any one of the preceding claims.

9. The drug delivery device according to claim 8, wherein the barrel (32) is adapted to engage with a removable cap (18) designed to cover and/or to protect the cartridge (30).

10. The drug delivery device according to any one of the preceding claims 8 or 9, wherein the barrel (32) comprises at least one annular recess (46) and/or at least one annular protruding portion (34) at its outer periphery.

11. The drug delivery device according to any one of the preceding claims 8 to 10, wherein the cartridge (30) comprises a threaded portion (36) near its distal end to engage with a needle assembly.

12. The drug delivery device according to any one of the preceding claims 8 to 11, wherein the interface (48) of cartridge (30) and body (12) is adapted to provide a releasable or non-releasable interconnection of cartridge (30) and body (12).

13. The drug delivery device according to any one of the preceding claims 8 to 12, wherein the barrel (32) is an integral part of a housing of the device.

14. The drug delivery device according to any one of the preceding claims 8 to 13, wherein the drive mechanism (25) is adapted to set and/or to dispense a predefined dose of the medicament.

## Patentansprüche

1. Kartusche für eine zum Einstellen und Abgeben einer Dosis eines Arzneimittels ausgestaltete Medikamenten-Verabreichungsvorrichtung, welche ein ein Gehäuse (12) zur Unterbringung eines Antriebsmechanismus (25) mit einer Kolbenstange (27) ausweist, wobei die Kartusche Folgendes umfasst:
- einen Zylinder (32), der durch einen Kolben (42) abgedichtet ist, welcher entlang einer axialen Richtung (11, 13) darin gleitend verschiebbar ist,
- einen Verbindungabschnitt (48) neben einem proximalen Ende zum Eingriff mit einem Gehäuse (12) der Medikamenten-Verabreichungsvorrichtung (10), und
- wobei der Zylinder (32) an seinem Außenumfang wenigstens einen sich radial erstreckenden Abschnitt (34) aufweist, welcher zum Zusammenwirken mit wenigstens einer weiteren funktionellen Komponente (30) der Vorrichtung ausgestaltet ist, und
- wobei der sich radial erstreckende Abschnitt (34) einen ringförmigen Vorsprung umfasst, der abgeflacht ist und eine Außenseite (35) aufweist, die sich parallel zur Längsachse (31) der Kartusche erstreckt, **dadurch gekennzeichnet, dass** der sich radial erstreckende Abschnitt in einem mittleren Abschnitt des Zylinders bereitgestellt ist.

2. Kartusche nach Anspruch 1, wobei der Verbindungsabschnitt einen Bajonettverschlussmechanismus und/oder eine Arretierungsanordnung umfasst.

3. Kartusche nach Anspruch 1 oder 2, wobei der Zylinder (32) wenigstens eine ringförmige Aussparung (46) und/oder wenigstens einen ringförmigen hervorstehenden Abschnitt (34) an seinem Außenumfang umfasst.

4. Kartusche nach Anspruch 3, wobei der ringförmige hervorstehende Abschnitt eine periphere Stützfläche umfasst, um eine wechselseitige periphere Anlage zwischen einer Vielzahl von Kartuschen zu ermöglichen, die dicht in oder auf einem Transportbehälter angeordnet sind.

5. Kartusche nach einem der vorhergehenden Ansprüche 2 bis 4, wobei der Kolben (42) in axialer Richtung (11, 13) eine symmetrische Gestalt aufweist.

6. Kartusche nach einem der vorhergehenden Ansprüche 2 bis 5, wobei der Zylinder (32) aus einem Kunststoffmaterial gefertigt ist.

7. Kartusche nach einem der vorhergehenden Ansprüche 2 bis 6, wobei der Verbindungsabschnitt (48) ausgelegt ist, um eine zerstörungsfreie und/oder zerstörende Verbindung zwischen der Kartusche (30) und dem Gehäuse (12) bereitzustellen.

8. Medikamenten-Verabreichungsvorrichtung zum Einstellen und Abgeben einer Dosis eines Arzneimittels, umfassend:
- ein Gehäuse (12) zur Unterbringung eines Antriebsmechanismus (25) mit einer Kolbenstange (27), und
- eine Kartusche (30) nach einem der vorhergehenden Ansprüche.

9. Medikamenten-Verabreichungsvorrichtung nach Anspruch 8, wobei der Zylinder (32) zum Zusammenwirken mit einer abnehmbaren Kappe (18) zur Abdeckung und/oder zum Schutz der Kartusche (30) ausgebildet ist.

10. Medikamenten-Verabreichungsvorrichtung nach einem der vorhergehenden Ansprüche 8 oder 9, wobei der Zylinder (32) wenigstens eine ringförmige Aussparung (46) und/oder wenigstens einen ringförmigen hervorstehenden Abschnitt (34) an seinem Außenumfang umfasst.

11. Medikamenten-Verabreichungsvorrichtung nach einem der vorhergehenden Ansprüche 8 bis 10, wobei die Kartusche (30) einen Gewindeabschnitt (36) neben ihrem distalen Ende zum Eingriff mit einer Nadelanordnung umfasst.

12. Medikamenten-Verabreichungsvorrichtung nach einem der vorhergehenden Ansprüche 8 bis 11, wobei der Verbindungsabschnitt (48) der Kartusche (30) und des Gehäuses (12) dazu ausgelegt ist, eine lösbare oder nicht lösbare Verbindung von Kartusche (30) und Gehäuse (12) bereitzustellen.

13. Medikamenten-Verabreichungsvorrichtung nach einem der vorhergehenden Ansprüche 8 bis 12, wobei der Zylinder (32) ein einstückiger Bestandteil eines Gehäuses der Vorrichtung ist.

14. Medikamenten-Verabreichungsvorrichtung nach einem der vorhergehenden Ansprüche 8 bis 13, wobei der Antriebsmechanismus (25) dazu ausgelegt ist, eine vordefinierte Dosis des Arzneimittels einzustellen und/oder abzugeben.

## Revendications

1. Cartouche pour un dispositif d'administration de médicament destinée à régler et distribuer une dose d'un médicament et comportant un corps (12) pour loger un mécanisme (25) d'entraînement ayant une tige (27) de piston, la cartouche comportant :
- un cylindre (32) scellé de manière étanche par un piston (42) déplacé de manière coulissante dans celui-ci le long d'une direction axiale (11, 13),
- une partie (48) interface près d'une extrémité proximale pour se mettre en prise avec un corps (12) du dispositif (10) d'administration de médicament, et
- dans laquelle le cylindre (32) comporte au moins une partie (34) s'étendant radialement au niveau de sa périphérie externe pour coopérer avec au moins un autre composant (30) fonctionnel du dispositif, et
- la partie (34) s'étendant radialement comportant une saillie annulaire qui est aplatie et comporte une surface (35) externe qui s'étend parallèlement à l'axe (31) longitudinal de la cartouche, **caractérisée en ce que** la partie s'étendant radialement est fournie dans une section centrale du cylindre.

2. Cartouche selon la revendication 1, dans laquelle l'interface comporte un mécanisme de loquet à baïonnette et/ou un ensemble verrou.

3. Cartouche selon la revendication 1 ou 2, dans laquelle le cylindre (32) comporte au moins un renfoncement (46) annulaire et/ou au moins une partie (34) faisant saillie annulaire au niveau de sa périphérie externe.

4. Cartouche selon la revendication 3, dans laquelle la partie saillante annulaire comporte une face de support périphérique pour permettre un appui périphérique mutuel entre une pluralité de cartouches disposées de manière dense dans ou sur un contenant de transport.

5. Cartouche selon l'une quelconque des revendications précédentes 2 à 4, dans laquelle le piston (42) a une forme symétrique dans une direction (11, 13) axiale.

6. Cartouche selon l'une quelconque des revendications précédentes 2 à 5, dans laquelle le cylindre (32) est constitué d'un matériau en plastique.

7. Cartouche selon l'une quelconque des revendications précédentes 2 à 6, dans laquelle l'interface (48) est apte à fournir une liaison non destructive et/ou destructive entre la cartouche (30) et le corps (12).

8. Dispositif d'administration de médicament destiné à régler et à distribuer une dose d'un médicament, comportant :
- un corps (12) pour loger un mécanisme (25) d'entraînement ayant une tige (27) de piston, et
- une cartouche (30) selon l'une quelconque des revendications précédentes.

9. Dispositif d'administration de médicament selon la revendication 8, dans lequel le cylindre (32) est apte à se mettre en prise avec un capuchon (18) amovible conçu pour recouvrir et/ou pour protéger la cartouche (30).

10. Dispositif d'administration de médicament selon l'une quelconque des revendications précédentes 8 ou 9, dans lequel le cylindre (32) comporte au moins un renfoncement (46) annulaire et/ou au moins une partie (34) faisant saillie annulaire au niveau de sa périphérie externe.

11. Dispositif d'administration de médicament selon l'une quelconque des revendications précédentes 8 à 10, dans lequel la cartouche (30) comporte une partie (36) filetée près de son extrémité distale pour se mettre en prise avec un ensemble aiguille.

12. Dispositif d'administration de médicament selon l'une quelconque des revendications précédentes 8 à 11, dans lequel l'interface (48) de la cartouche (30) et du corps (12) est apte à fournir une liaison libérable ou non libérable de la cartouche (30) et du corps (12).

13. Dispositif d'administration de médicament selon l'une quelconque des revendications précédentes 8 à 12, dans lequel le cylindre (32) fait partie intégrante d'un logement du dispositif.

14. Dispositif d'administration de médicament selon l'une quelconque des revendications précédentes 8 à 13, dans lequel le mécanisme (25) d'entraînement est apte à régler et/ou administrer une dose prédéfinie du médicament.
